# EUROPEAN PATENT APPLICATION

(11) **EP 4 141 844 A1**
(43) Date of publication of application: **01.03.2023**
(21) Application number: 21793802.6
(22) Date of filing: 20.04.2021
(51) Int. Cl.: G09B 9/00, G06Q 50/10, G06Q 50/20, G09B 23/28, A61B 34/10, A61B 34/00, A61B 90/00

(54) **SURGICAL SKILL TRAINING SYSTEM AND MACHINE LEARNING-BASED SURGICAL GUIDE SYSTEM USING THREE-DIMENSIONAL IMAGING**

(30) Priority: 21.04.2020 KR 20200048314
(71) Applicant: Samsung Life Public Welfare Foundation, Seoul 04348 (KR)
(72) Inventor: CHOE, Jun Ho, Seongnam-si, Gyeonggi-do 13582 (KR)
(74) Representative: Patentanwälte Olbricht Buchhold Keulertz
(86) International application number: PCT/KR2021/004960
(87) International publication number: WO 2021/215800

(57) **Abstract**

A surgical skill training system includes: a data collecting unit configured to collect actual surgical skill data on a patient of an operating surgeon; an image providing server configured to generate a 3-dimensional (3D) surgical image for surgical skill training, based on the actual surgical skill data; and a user device configured to display the 3D surgical image, wherein the image providing server includes: a patient image generating unit configured to generate a patient image, based on patient information of the patient; a surgical stage classifying unit configured to classify the actual surgical skill data into actual surgical skill data for each surgical stage performed by the operating surgeon; and a 3D image generating unit configured to generate the 3D surgical image by using the patient image, and feature information detected from the actual surgical skill data.

## Description

### TECHNICAL FIELD

The present disclosure relates to a surgical skill training system and machine learning-based surgical guide system, which use a 3-dimensional image.

### BACKGROUND ART

A surgical operation requires a high degree of skill, and thus a surgical process is highly dependent on accumulated know-hows and practiced skills of an operating surgeon. Accordingly, surgical skill training, simulation, and a guide system for transferring surgical know-hows of skilled surgeons for training are required.

An existing surgical skill training system is executed only at a surgical scene around an operating surgeon conducting a surgery on a patient, and thus there have been temporal and spatial restrictions on apprenticeship education, for example, the number or sights of trainees provided with accurate surgical skill information are limited.

Meanwhile, surgical skills of operating surgeons are different regarding patients for each of various surgical diseases, and thus there have been restrictions on successful rates of surgeries and security assurances for various cases. To maximize such successful rates and security of surgeries, training and simulation systems not only for direct surgical movements on a surgical site, but also for an entire surgical process including pre- and post-preparation and a finishing stage are required.

Also, in the prior art, surgical image information of an operating surgeon is used for surgical skill training as it is, and thus it is difficult to provide training in which various surgical skills are accurately and objectively evaluated and verified.

### DESCRIPTION OF EMBODIMENTS

### TECHNICAL PROBLEM

Embodiments of the present disclosure provide a surgical skill training/simulation system wherein a limitation on apprenticeship education and temporal and spatial restrictions may be overcome by using a 3-dimensional image generated based on surgical images of an entire surgical process of an operating surgeon.

Also, embodiments of the present disclosure provide an artificial intelligence (Al)-based surgical guide system for various surgical environments according to a user request by constructing a learning model in which surgical images of a plurality of operating surgeons are categorized.

### SOLUTION TO PROBLEM

A surgical skill training system according to an embodiment of the present disclosure includes: a data collecting unit configured to collect actual surgical skill data on a patient of an operating surgeon; an image providing server configured to generate a 3-dimensional (3D) surgical image for surgical skill training, based on the actual surgical skill data; and a user device configured to display the 3D surgical image, wherein the image providing server includes: a patient image generating unit configured to generate a patient image, based on patient information of the patient; a surgical stage classifying unit configured to classify the actual surgical skill data into actual surgical skill data for each surgical stage performed by the operating surgeon; and a 3D image generating unit configured to generate the 3D surgical image by using the patient image, and feature information detected from the actual surgical skill data.

The actual surgical skill data for each surgical stage may include first surgical skill data performed during a preparation stage, second surgical skill data performed during an intermediate stage after the preparation stage, and third surgical skill data performed during a finishing stage after the intermediate stage, and the surgical stage classifying unit may classify the first surgical skill data, the second surgical skill data, and the third surgical skill data according to first surgical movement information of the operating surgeon.

The image providing server may further include: a feature information detecting unit configured to detect the feature information for each of the first surgical skill data, the second surgical skill data, and the third surgical skill data; and an image signal generating unit configured to generate an image signal based on the feature information, and the 3D image generating unit may generate the 3D surgical image by combining the image signal with the patient image, wherein the 3D surgical image may include a first surgical image generated based on the first surgical skill data, a second surgical image generated based on the second surgical skill data, and a third surgical image generated based on the third surgical skill data.

The first surgical skill data may include surgical instrument information, patient posture information, and surgical assistive instrument information, the second surgical skill data may include surgical route information and surgical movement information, and the third surgical skill data may include suture route information, suture method information, and residue information indicating whether the surgical instrument or surgical assistive instrument remains in a human body.

The data collecting unit may include a data collecting apparatus configured to obtain the actual surgical skill data by being worn on the operating surgeon, and the data collecting apparatus may include a detecting unit configured to obtain tactile information of the actual surgical skill data by using a sensor, and a photographing unit configured to obtain visual information of the actual surgical skill data by using an optical element.

The user device may include: a communication unit configured to receive the 3D surgical image; an interface unit configured to receive a user request of selecting the patient information or surgical stage; and a display unit configured to display the 3D surgical image, wherein the patient information may include a disease type, gender, age, height, weight, body mass index (BMI), period of illness, and underlying disease of the patient.

A machine learning-based surgical guide system according to an embodiment of the present disclosure includes: a data collecting unit configured to collect a plurality of pieces of actual surgical skill data on patients of a plurality of operating surgeons; an image providing server including a learning model trained based on the plurality of pieces of actual surgical skill data, and a 3-dimensional image generating unit configured to generate a 3D surgical guide image based on a selected image output by the learning model; and a user device configured to display the 3D surgical guide image, wherein the learning model classifies the plurality of pieces of actual surgical skill data according to surgery item information including patient information and surgical stage information, and outputs the selected image according to a user input of selecting a surgical condition.

The learning model may categorize the plurality of pieces of actual surgical skill data by using a patient information recognizing unit, a surgical instrument recognizing unit, a surgical stage classifying unit, a movement information detecting unit, and a successful rate evaluating unit.

The plurality of pieces of actual surgical skill data may include first surgical skill data performed during a preparation stage, second surgical skill data performed during an intermediate stage after the preparation stage, and third surgical skill data performed during a finishing stage after the intermediate stage, and the surgical stage classifying unit of the learning model may classify the first surgical skill data, the second surgical skill data, and the third surgical skill data according to first surgical movement information of the plurality of operating surgeons.

The image providing server may include: a feature information detecting unit configured to detect feature information of surgical skill data included in the selected image; and an image signal generating unit configured to generate an image signal corresponding to the feature information.

The 3D image generating unit may generate the 3D surgical guide image by combining the image signal with the selected image, wherein the 3D surgical guide image may include a first surgical image generated based on the first surgical skill data, a second surgical image generated based on the second surgical skill data, and a third surgical image generated based on the third surgical skill data.

The successful rate evaluating unit may classify the plurality of pieces of actual surgical skill data into surgery success data and surgery failure data by detecting successful rates of surgeries.

### ADVANTAGEOUS EFFECTS OF DISCLOSURE

According to embodiments of the present disclosure, a limitation on apprenticeship education and temporal and spatial restrictions can be overcome through surgical skill training/simulation system using a 3-dimensional image generated based on actual surgical skill data on an entire surgical process of an operating surgeon.

Also, an artificial intelligence (Al)-based surgical guide system for various surgical environments according to a user request can be provided by constructing a learning model in which surgical images of a plurality of operating surgeons are categorized.

### BRIEF DESCRIPTION OF DRAWINGS

FIG. 1 is a diagram schematically showing a configuration of a surgical skill training system, according to an embodiment of the present disclosure.
FIG. 2 is a diagram schematically showing a configuration of a data collecting unit, according to an embodiment of the present disclosure.
FIG. 3 is a diagram showing a configuration of a control unit of an image providing server, according to an embodiment of the present disclosure.
FIG. 4 is a flowchart of operations of a surgical skill training system, according to an embodiment of the present disclosure.
FIG. 5 is a diagram schematically showing a configuration of a machine learning-based surgical guide system, according to an embodiment of the present disclosure.
FIG. 6 is a diagram for describing operations of a learning model, according to an embodiment of the present disclosure.
FIG. 7 is a flowchart of operations of a surgical guide system, according to an embodiment of the present disclosure.
FIG. 8 illustrates an example of a 3-dimensional (3D) surgical image output by a surgical guide system, according to an embodiment of the present disclosure.
FIG. 9 is a diagram showing a data collecting apparatus according to an embodiment of the present disclosure.

### MODE OF DISCLOSURE

The present disclosure may have various modifications and various embodiments, and specific embodiments are illustrated in the drawings and are described in detail in the detailed description. Effects and features of the present disclosure and methods of achieving the same will become apparent with reference to embodiments described in detail with reference to the drawings. However, the present disclosure is not limited to the embodiments described below, and may be implemented in various forms.

Hereinafter, embodiments will be described in detail with reference to the accompanying drawings, and in the following description with reference to the drawings, like reference numerals refer to like elements and redundant descriptions thereof will be omitted.

In the following embodiments, the terms "first" and "second" are not used in a limited sense and are used to distinguish one component from another component. In the following embodiments, an expression used in the singular encompasses the expression of the plural, unless it has a clearly different meaning in the context. In the following embodiments, it will be further understood that the terms "comprise" and/or "comprising" used herein specify the presence of stated features or components, but do not preclude the presence or addition of one or more other features or components. In the drawings, for convenience of description, sizes of components may be exaggerated or reduced. For example, because sizes and shapes of components in the drawings are arbitrarily illustrated for convenience of explanation, the present disclosure is not necessarily limited thereto.

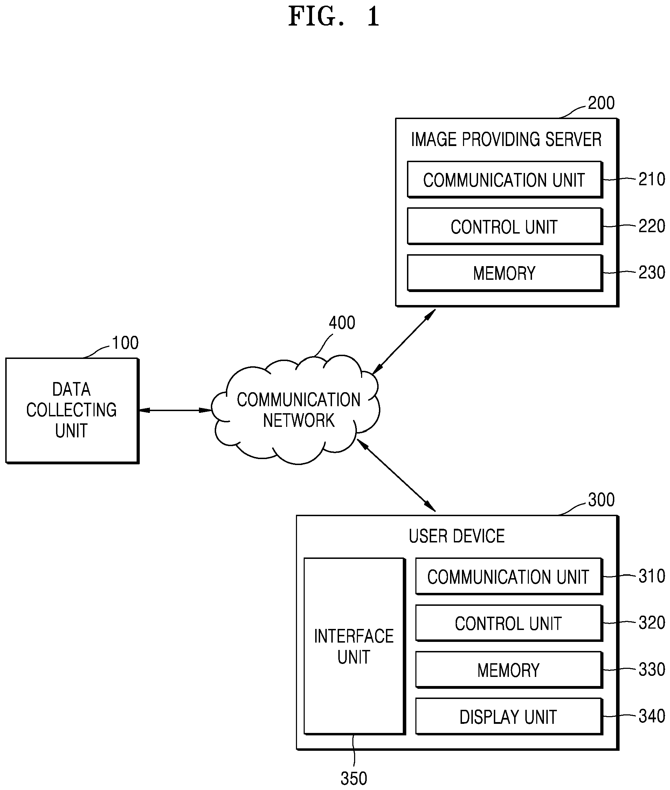

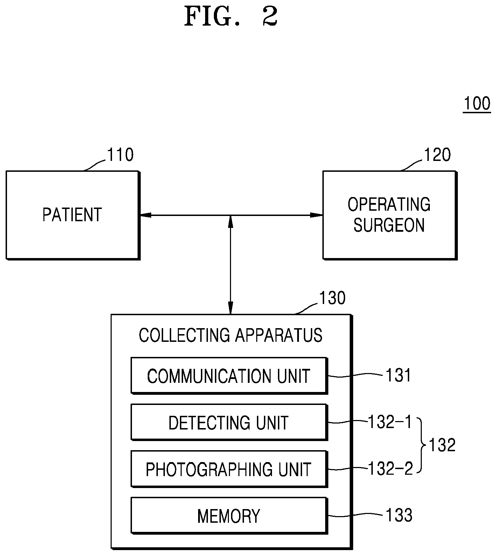

FIG. 1 is a diagram schematically showing a configuration of a surgical skill training system, according to an embodiment of the present disclosure, and FIG. 2 is a diagram schematically showing a configuration of a data collecting unit, according to an embodiment of the present disclosure.

The surgical skill training system includes a data collecting unit 100, an image providing server 200, and a user device 300, and the data collecting unit 100, the image providing server 200, and the user device 300 may communicate with each other through a communication network 400.

The data collecting unit 100, the image providing server 200, and the user device 300 may respectively include communication units 131, 210, and 310, control units 220, and 320, and memories 133, 230, and 330. The communication units 131, 210, and 310 may communicate with various types of external devices and servers, according to various types of communication methods. The communication units 131, 210, and 310 of the present disclosure may transmit and receive data to and from each other by being connected to each other by the communication network 400. Here, the communication network 400 may be a wireless communication network, and for example, may be a 5th generation (5G) communication network having high-speed and low-latency characteristics, but is not limited thereto.

The control units 220, and 320 may perform operations of controlling apparatuses or servers (the data collecting unit 100, the image providing server 200, and the user device 300) including the memories 133, 230, and 330 by using various programs and data stored in the memories 133, 230, and 330, respectively. The control units 220, and 320 may include any type of apparatuses for processing data, such as a processor. The processor may include a processing device, such as a microprocessor, a central processing unit (CPU), a processor core, a multiprocessor, an application-specific integrated circuit (ASCI), or a field programmable gate array (FPGA), but is not limited thereto.

The memories 133, 230, and 330 perform a function of temporarily or permanently storing data processed by the apparatuses or servers (the data collecting unit 100, the image providing server 200, and the user device 300) including the memories 133, 230, and 330, respectively. The memories 133, 230, and 330 may each include a permanent mass storage device, such as a random access memory (RAM), a read-only memory (ROM), or a disk drive, but the scope of the present disclosure is not limited thereto.

The memory 230 of the image providing server 200 may store all pieces of data required to generate a 3-dimensional (3D) surgical image. For example, the memory 230 may include a patient information storage unit (not shown) storing patient information, and surgical instrument information storage unit (not shown) storing surgical instrument information used for each type of surgery. The memory 330 of the user device 300 may also store patient information and surgical instrument information.

Referring to FIG. 2 together, the data collecting unit 100 collects actual surgical skill data regarding a patient 110 of an operating surgeon 120, and may include the patient 110, the operating surgeon 120, and a data collecting apparatus 130. The actual surgical skill data includes various types of data related to surgical movement actually performed by the operating surgeon 120 on the patient 110, and may include various types of surgical skill information related to a surgical image, a surgical route, a surgical instrument, a surgical site, a state of the surgical site (a damage level, a metastasis level, a color, or the like), and strength or depth of surgical movement, which are actually practiced.

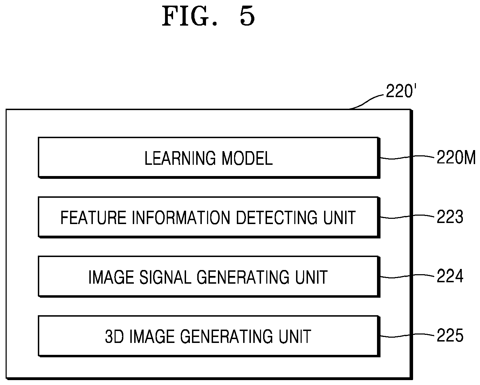

In FIG. 2, one patient 110 and one operating surgeon 120 are illustrated, but the actual surgical skill data collected by the data collecting unit 100 may be collected from surgeries performed by the plurality of operating surgeons 120 on the different patients 110. This will be described in detail with reference to embodiments of FIGS. 5 through 7 described below.

The data collecting apparatus 130 is worn on the operating surgeon 120 to obtain the actual surgical skill data, and may be a wearable collecting apparatus that may be easily worn by the operating surgeon 120. The data collecting apparatus 130 may include the communication unit 131, the memory 133, and the data obtaining unit 132, and the data obtaining unit 132 may include a detecting unit 132-1 and a photographing unit 132-2.

The detecting unit 132-1 may obtain tactile information of the actual surgical skill data by using a sensor. The detecting unit 132-1 may be a surgical glove including the sensor. The sensor may include various sensors for easily obtaining the tactile information of the actual surgical skill data by the operating surgeon 120. The sensor may measure movements of a hand or fingers of the operating surgeon 120 and output signals corresponding to different movements. For example, the sensor may include a pressure sensor, an inertial measurement unit (IMU), and the like. The pressure sensor measures contact information and strength information of hand movement of the operating surgeon 120, and the IMU measures rotation information or speed information of the hand movement by including a gyroscope, an accelerometer, and a magnetic field sensor, and thus 3D information of the surgical movement may be determined.

For example, at least one of the gyroscope (not shown), the accelerometer (not shown), and the magnetic field sensor (not shown) may be a 3-axis sensor. The gyroscope may measure rotational inertia and/or rotation rate (for example, deg/sec as a unit) using an angular speed of the hand movement of the operating surgeon 120, the accelerometer may measure movement inertia (for example, g (1 g = 9.8 m/s²) as a unit) of the hand of the operating surgeon 120 using acceleration of the hand movement of the operating surgeon 120, and the magnetic field sensor may measure an azimuth of the hand movement of the operating surgeon 120.

The type of sensor is not limited thereto, and the sensor may include various combinations such that 3D information, such as a location, rotation, movement information, of the surgical movement of the operating surgeon 120 is easily determined. A location of the sensor also does not limit the present disclosure.

The photographing unit 132-2 may obtain visual information of the actual surgical skill data by using an optical element. The photographing unit 132-2 may be a wearable device that is easily worn on the operating surgeon 120, and for example, may be a head-mounted display (HMD) that may be worn on the head of the operating surgeon 120. The photographing unit 132-2 may include a camera and various sensors such that actual surgical skill data with depth may be obtained while capturing the surgical image of the actual surgical skill data. The HMD may have binocular vision such that the 3D surgical image based on the actual surgical skill data has a 3D effect and depth. The 3D surgical image may be a virtual reality (VR) image, an augmented reality (AR) image, or a mixed reality (MR) image.

Also, the photographing unit 132-2 may further include a voice recognition unit (not shown) and perform photographing by recognizing a voice command related to a viewpoint, angle, and the like for capturing a surgical image of the operating surgeon 120.

In FIG. 2, the communication unit 131 is illustrated as a separate component from the detecting unit 132-1 and the photographing unit 132-2, but the communication unit 131 may be included in each of the detecting unit 132-1 and the photographing unit 132-2 as individual components.

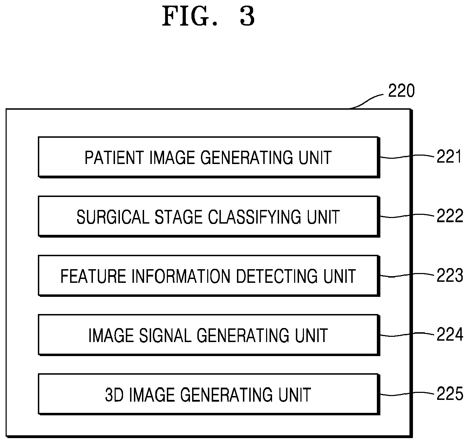

Referring back to FIG. 1, the image providing server 200 generates the 3D surgical image for surgical skill training, based on the actual surgical skill data, and will be described below with reference to FIG. 3 together. FIG. 3 is a diagram showing a configuration of the control unit 220 of the image providing server 200, according to an embodiment of the present disclosure.

The control unit 220 of the image providing server 200 may include a patient image generating unit 221, a surgical stage classifying unit 222, a feature information detecting unit 223, an image signal generating unit 224, and a 3D image generating unit 225.

The patient image generating unit 221 may generate a patient image based on patient information of the patient 110. The patient image may be an actual patient image captured through the photographing unit 132-2 or may be a VR image regenerated by the patient image generating unit 221 based on the patient information.

The surgical stage classifying unit 222 may classify the actual surgical skill data according to surgical stages performed by the operating surgeon 120. The actual surgical skill data for each surgical stage includes first surgical skill data performed during a surgery preparation stage, second surgical skill data performed during a surgery intermediate stage after the surgery preparation stage, and third surgical skill data performed during a surgery finishing stage after the surgery intermediate stage.

The first surgical skill data may include all pieces of data required for the surgery preparation stage before the real surgery intermediate stage is performed. For example, the first surgical skill data may include surgical instrument information, patient posture information, surgical assistive instrument information, and surgery atmosphere information. Surgical instruments of the surgical instrument information may be various surgical instruments required for a surgical operation, and for example, may include a laparoscopic system required during a laparoscopic surgery, a dissector, a retractor, and a grasper, but are not limited thereto. The patient posture information is information about a patient posture that maximizes a successful rate of each surgery type, and may include angles and arrangement postures of parts of a body. The surgical assistive instrument information may include information about arrangements of a surgical antiseptic cloth, a draping cloth, and the like. The surgery atmosphere information may include illumination, brightness, temperature, and the like of an operating room.

The second surgical skill data may include all pieces of data obtained during the surgery intermediate stage during which a real surgical movement is performed. For example, the second surgical skill data may include surgical route information and surgical movement information. For example, when the surgical movement is incision, the surgical route information may include incision position information and incision route information according to the incision position information, and the surgical movement information may include incision depth, angle, and strength information.

The third surgical skill data may include all pieces of data obtained during the surgery finishing stage. For example, the third surgical skill data may include suture route information, suture method information, and residue information indicating whether the surgical instrument or surgical assistive instrument remains in a human body.

The surgical stage classifying unit 222 may classify the first surgical skill data, the second surgical skill data, and the third surgical skill data according to a pre-set criterion related to the first surgical movement information of the operating surgeon 120. The first surgical movement information is a criterion for classifying the actual surgical skill data, and may include a range of surgical route, a speed of surgical movement, and the like. For example, when the range of surgical route exceeds a certain range, the actual surgical skill data may be classified as the first surgical skill data, and when the range of surgical route is equal to or less than the certain range, the actual surgical skill data may be classified as the second surgical skill data.

The feature information detecting unit 223 may detect feature information of the surgical movement with respect to each of the first surgical skill data, second surgical skill data, and third surgical skill data classified by the surgical stage classifying unit 222. The feature information may include various types of surgical information about a surgical route, a surgical instrument, a surgical site, a state (a damage level, a metastasis level, a color, or the like) of the surgical site, and strength or depth of surgical movement.

The image signal generating unit 224 may generate an image signal based on the feature information. The image signal may be any type of image signal for implementing VR, AR, or MR in combination with the patient image, and for example, may be realized in a form of user interface (Ul) or pop-up image.

The 3D image generating unit 225 may generate a 3D surgical image by using the patient image and the feature information detected from the actual surgical skill data. The 3D image generating unit 225 may generate the 3D surgical image by combining the image signal with the patient image, wherein the 3D surgical image may include a first surgical image generated based on the first surgical skill data, a second surgical image generated based on the second surgical skill data, and a third surgical image generated based on the third surgical skill data. In other words, the first surgical image may be an image in which the image signal generated based on the first surgical skill data is combined with the patient image, the second surgical image may be an image in which the image signal generated based on the second surgical skill data is combined with the patient image, and the third surgical image may be an image in which the image signal generated based on the third surgical skill data is combined with the patient image.

Referring back to FIG. 1, the user device 300 may display the 3D surgical image generated by the image providing server 200.

In detail, the user device 300 includes the communication unit 310, the control unit 320, and the memory 330, and the communication unit 310 may receive data on the 3D surgical image. The user device 300 may include a display unit 340 and an interface unit 350.

The display unit 340 may display the 3D surgical image to provide, to a user, surgical skill training using a 3D image.

The interface unit 350 may receive a user request of selecting patient information or surgical stage, and the communication unit 310 may transmit, to the image providing server 200, the user request received through the interface unit 350. The patient information may include a disease type, gender, age, height, weight, body mass index (BMI), period of illness, and underlying disease of a patient.

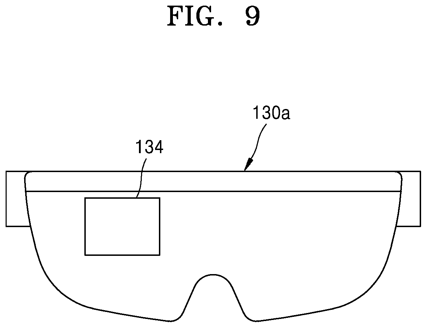

The user device 300 may be any type of device by which a user receives a training service through the 3D surgical image. For example, the user device 300 may be a wearable device, such as a smart watch, smart glasses, or HMD, capable of exchanging data (or interworking) with the data collecting unit 100 and image providing server 200. Alternatively, the user device 300 may be a mobile device, such as a smart phone or a tablet personal computer (PC), but is not limited thereto. According to an embodiment, the user device 300 may be the data collecting apparatus 130 worn by the operating surgeon 120 such that a surgery simulation service is provided, and at this time, the 3D surgical image may be displayed on an image display unit 134 (see FIG. 9) of the data collecting apparatus 130.

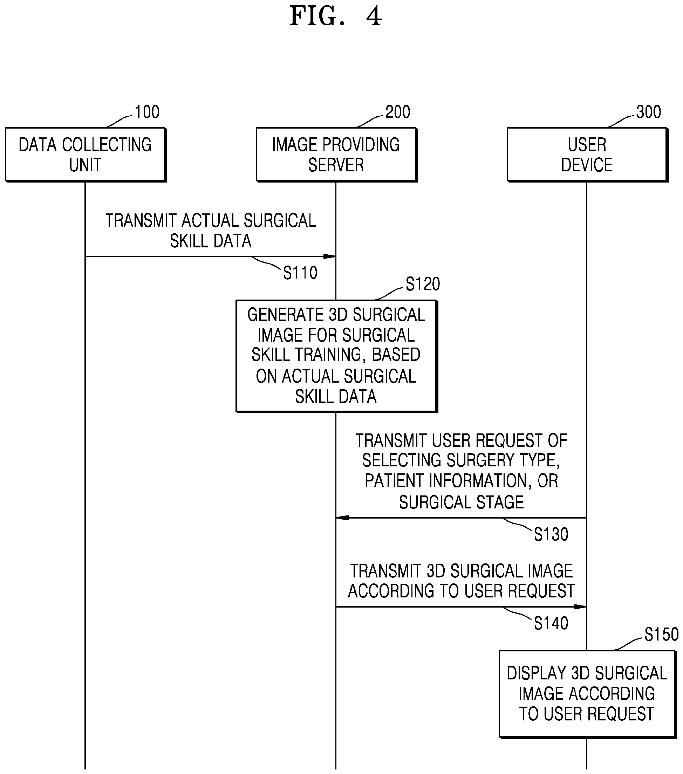

FIG. 4 is a flowchart of operations of a surgical skill training system, according to an embodiment of the present disclosure.

The data collecting unit 100 may obtain the actual surgical skill data and transmit the same to the image providing server 200 (operation S110). The image providing server 200 may generate the 3D surgical image for surgical skill training, based on the actual surgical skill data received from the data collecting unit 100 (operation S120). Here, the 3D surgical image includes surgical images for an entire surgical stage including surgery preparation, intermediate, and finishing stages, and thus surgical skill information on not only a real surgery stage, but also all surgery processes before and after the real surgery stage may be provided and accordingly, a successful rate and stability of a surgery may be increased.

The user device 300 may receive a user request of selecting a surgery type, patient information, or surgical stage, and transmit the same to the image providing server 200 (operation S130). The image providing server 200 may output the 3D surgical image according to the user request and transmit the same to the user device 300 again (operation S140). The user device 300 may display the 3D surgical image according to the user request to provide a surgical skill training/simulation service (operation S150).

As such, according to embodiments of the present disclosure, a surgical skill training/simulation system may be provided by using a 3D image generated based on the actual surgical skill data of the entire surgical process of the operating surgeon 120, and accordingly, limitation of apprenticeship education and temporal and spatial restrictions may be overcome.

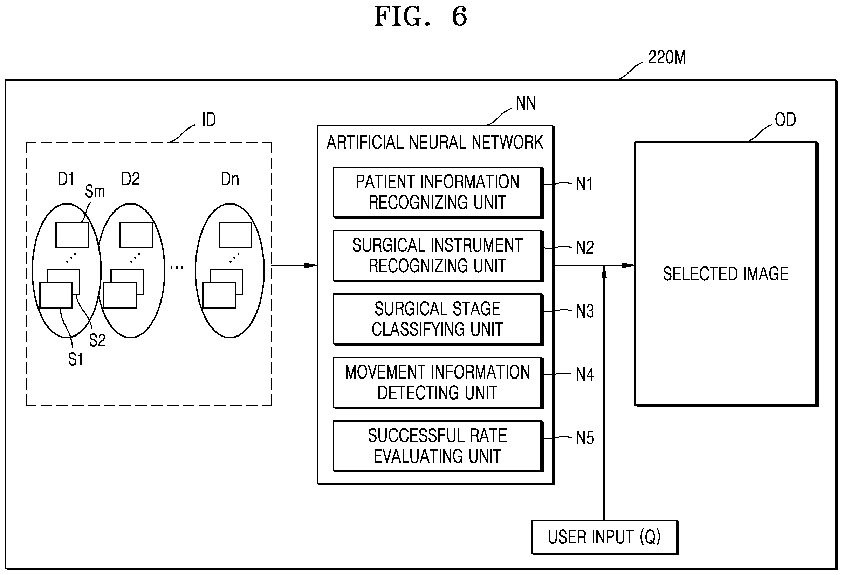

FIG. 5 is a diagram schematically showing a configuration of a machine learning-based surgical guide system, according to an embodiment of the present disclosure, and FIG. 6 is a diagram for describing operations of a learning model, according to an embodiment of the present disclosure. Hereinafter, descriptions overlapping those of the above embodiments may be made brief or omitted, and features distinguished from the above embodiments will be mainly described together with reference to FIGS. 1 through 3.

The machine learning-based surgical guide system may include the data collecting unit 100, image providing server 200, and user device 300, as shown in FIG. 1. The data collecting unit 100 may collect actual surgical skill data of the plurality of operating surgeons 120 on the patients 110. In other words, when one operating surgeon 120 and one patient 110 are one group, the data collecting unit 100 may collect the actual surgical skill data of several groups.

The image providing server 200 may include a control unit 220' according to an embodiment of FIG. 5. The control unit 220' may include a learning model 220M, the feature information detecting unit 223, the image signal generating unit 224, and the 3D image generating unit 225, and same descriptions as those of FIG. 3 may be applied to the feature information detecting unit 223, image signal generating unit 224, and 3D image generating unit 225.

The learning model 220M may be trained based on the actual surgical skill data, and a plurality of pieces of actual surgical skill data may be classified according to various types of surgery item information including patient information and surgical stage information. Also, the learning model 220M may output a selected image according to a user input of selecting a surgical condition.

In detail, referring to FIG. 6 together, a plurality of pieces of actual surgical skill data of a plurality of operating surgeons D1 through Dn (wherein n is a natural number greater than 1) on a plurality of patients are input, as input data ID, to an artificial neural network NN of the learning model 220M, and the learning model 220M may be trained based on the plurality of pieces of actual surgical skill data. For example, when a first operating surgeon D1 performed surgeries on different m patients or performed m surgeries (wherein m is a natural number greater than 1), obtained pieces of actual surgical skill data may be S1 through Sm. Similarly, the input data ID may include a plurality of pieces of actual surgical skill data performed by different operating surgeons D2 through Dn.

Here, the artificial neural network NN may be trained after surgery types performed by the plurality of operating surgeons D1 through Dn are divided, or may be trained by receiving pieces of surgical skill data of various types of surgeries at once and also categorizing the surgery types.

The artificial neural network NN may categories the pieces of input data ID according to pre-set various surgery item standards, and construct a good quality surgical skill database in which know-hows of the plurality of operating surgeons 120 are integrated. The artificial neural network NN trained as such may output a selected image OD according to a user input Q of selecting various surgical conditions.

The input data ID may be categorized by a patient information recognizing unit N1, a surgical instrument recognizing unit N2, a surgical stage classifying unit N3, a movement information detecting unit N4, and a successful rate evaluating unit N5, which are pre-configured in the artificial neural network NN. The patient information recognizing unit N1 may detect and classify patient information of the input data ID, and the surgical instrument recognizing unit N2 may detect and classify surgical instrument information of the input data ID.

The surgical stage classifying unit N3 may detect and classify surgical stage information (one stage selected from among the surgery preparation/intermediate/finishing stages) of the input data ID. In detail, the plurality of pieces of actual surgical skill data may be classified into the first surgical skill data performed during the surgery preparation stage, the second surgical skill data performed during the surgery intermediate stage after the surgery preparation stage, and the third surgical skill data performed during the surgery finishing stage after the surgery intermediate stage. In other words, a large amount of input data ID may be classified according to surgical stages by the surgical stage classifying unit N3 of the learning model 220M.

The movement information detecting unit N4 may detect and classify information about surgical movements for surgical skill data for each surgical stage classified by the surgical stage classifying unit N3.

The successful rate evaluating unit N5 may detect a surgery successful rate of the input data ID according to a pre-determined rule, and classify the surgery successful rate as surgery success data or surgery failure data. For example, a specific numerical value for evaluating improvement of a disease of a patient before and after a surgery is assigned for each of the plurality of pieces of actual surgical skill data for measurement and comparison, and a weight is put depending on a difference before and after the specific numerical value to determine whether the surgery is successful or failed.

The user input Q may indicate selection on an item for providing a guide to a user with respect to various surgical condition, such as patient information, surgery type, surgical instrument, and surgical stage. Examples of the surgery type include laparoscopic appendix excision, thyroid surgery, breast cancer surgery, acute appendicitis, acute cholecystitis, perianal disease, and herniotomy, but are not limited thereto and include various surgeries. The learning model 220M may output the selected image OD corresponding to the surgical condition according to the user input Q, by using the artificial neural network NN trained as described above.

Referring back to FIG. 5, the feature information detecting unit 223 may detect feature information of surgical skill data included in the selected image OD output by the learning model 220M. The image signal generating unit 224 may generate an image signal corresponding to the feature information. The 3D image generating unit 225 may generate a 3D surgical image based on the selected image OD, wherein the 3D surgical image may be generated by combining the image signal with the selected image OD.

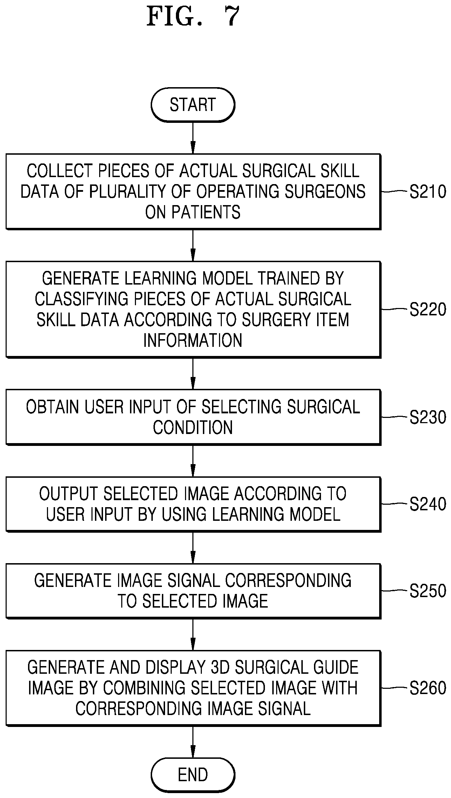

FIG. 7 is a flowchart of operations of a surgical guide system, according to an embodiment of the present disclosure.

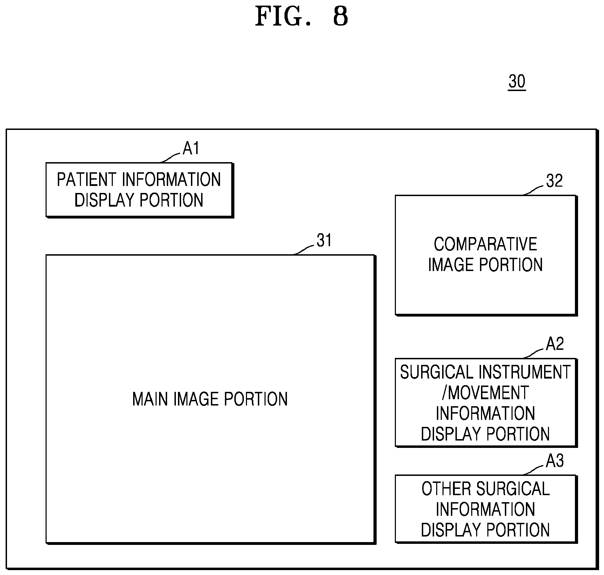

Pieces of actual surgical skill data of a plurality of operating surgeons on patients may be collected (S210). Then, a learning model trained by classifying the pieces of actual surgical skill data according to surgery item information may be trained and generated (S220). A user input of selecting a surgical condition may be obtained (S230). Then, a selected image according to the user input may be output by using the learning model (operation S240), and an image signal corresponding to the selected image may be generated (S250). Then, 3D surgical guide image may be generated and displayed by combining the selected image with the corresponding image signal (S260). The 3D surgical guide image corresponds to the 3D surgical image described above, but may be different from the 3D surgical image in that the 3D surgical guide image is a machine learning-based image generated by using the learning model 220M. The output 3D surgical guide image will be described in detail with reference to FIGS. 8 and 9 below.

As such, according to embodiments of the present disclosure, by constructing a learning model in which a massive amount of actual surgical skill data, in which know-hows of the plurality of operating surgeons 120 are integrated, are categorized according to various types of surgery item information, an artificial intelligence (Al)-based surgical guide system for various surgical conditions according to user requests may be provided.

Also, according to the machine learning-based surgical guide system according to embodiments of the present disclosure, a surgery simulation/guide service having improved accuracy and reliability may be provided by a learning model that is machine-trained based on pieces of actual surgical skill data of a plurality of operating surgeons.

FIG. 8 illustrates an example of a 3D surgical image 30 output by a surgical guide system, according to an embodiment of the present disclosure.

The 3D surgical image 30 may display a main image portion 31, a comparative image portion 32, and a plurality of Uls A1, A2, and A3. The main image portion 31 may reproduce a 3D surgical guide image for a first image, a second image, and a third image for surgical stages, respectively. The comparative image portion 32 may reproduce a surgical guide image of a case similar to a surgical condition according to a user request. The comparative image portion 32 may be displayed in a form of a pop-up window.

The first Ul A1 is a patient information display portion and may display various types of patient information described above. The second UI A2 is a surgical instrument/movement information display portion, and may display surgical instrument information when the 3D surgical guide image for the first image is reproduced in the main image portion 31, and display surgical movement information when the 3D surgical guide image for the second image is reproduced in the main image portion 31. The third UI A3 is another surgical information display portion, and may provide a guide by displaying various types of surgery item information required to perform a surgery, other than the patient information and the surgical instrument/movement information. Uls displayed in the image 30 are not limited to the Uls A1, A2, and A3 shown in FIG. 8, and display contents and locations may vary.

For example, a user may have requested a surgical guide for laparoscopic appendix excision (surgery type/disease selection) regarding a patient (patient information selection) with a specific gender, age, height, and BMI. In addition, when the user requests a surgical guide for a surgery preparation stage (surgical stage selection) of the surgery, the learning model 220M may provide a 3D surgical guide image according to the user input.

For example, the user may have requested the first image corresponding to the surgery preparation stage as the surgical stage. In this case, the image providing server 200 may generate and provide the 3D surgical guide image according to the user request. At this time, information on surgical instruments and a patient posture required for a specific surgery type may be guided. Then, information on arrangements of surgical assistive instrument, such as draping cloth and surgical antiseptic cloth, may be guided.

The user may have requested the second image corresponding to the surgery intermediate stage for the laparoscopic appendix excision as a surgical operation type. The image providing server 200 may guide an incision method according to a location (surgery region) of appendix, an incision location, and an incision route. For example, the image providing server 200 may guide surgical movements in an order of incision around a navel, fascia identification, clamping, incision, trocar insertion, carbon dioxide gas supply (insufflations), scope insertion, and surgery after remaining port insertion. It is obvious that detailed position, angle, and strength information may also be provided in relation to each surgical movement.

The learning model 220M according to an embodiment may identify whether there is data according to a specific user request, and when there is no data, provide data most similar to a surgical condition according to the specific user request by amending existing data.

The user device 300 (see FIG. 1) may further include a voice recognition unit and/or a voice output unit (not shown) to output voice data corresponding to various types of surgical information output through the 3D surgical guide image.

The 3D surgical guide image 30 may be displayed on the display unit 340 of the user device 300, and according to an embodiment, may be displayed on the image display unit of the data collecting apparatus 130.

FIG. 9 is a diagram showing a data collecting apparatus 130a according to an embodiment of the present disclosure. The data collecting apparatus 130a of FIG. 9 is illustrated as an HMD that is an example of the photographing unit 132-2. The 3D surgical guide image 30 of FIG. 8 may be displayed on the display unit 134. In other words, a machine learning-based surgical guide image according to an embodiment may be provided on a wearable collecting apparatus worn by the operating surgeon 120, in real time during a surgery. According to an embodiment, a surgical guide image may be displayed on the display unit 340 of the separate user device 300 such that Al-based surgical guide service is provided without temporal and spatial restrictions. Here, the user device 300 may be a wearable device worn on a trainee or may be any display device with a display function, such as a smart phone, a tablet PC, or a screen.

While the exemplary embodiments of the present disclosure have been illustrated and described above, the present disclosure is not limited to the specific embodiments described above, and various modifications may be possible by a person skilled in the art without departing from the gist of the present disclosure claimed in claims. These modifications should not be understood separately from the technical spirit or prospect of the present disclosure.

Therefore, the spirit of the present disclosure should not be determined limitedly based on the above-described embodiments, and not only the appended claims but also all ranges equivalent to or equivalently changed from the claims are within the scope of the spirit of the present disclosure.

## Claims

1. A surgical skill training system comprising:
a data collecting unit configured to collect actual surgical skill data on a patient of an operating surgeon;
an image providing server configured to generate a 3-dimensional (3D) surgical image for surgical skill training, based on the actual surgical skill data; and
a user device configured to receive patient information of a first patient and display the 3D surgical image,
wherein the image providing server comprises:
a patient image generating unit configured to generate a patient image, based on the patient information;
a surgical stage classifying unit configured to classify the actual surgical skill data into actual surgical skill data for each surgical stage performed by the operating surgeon; and
a 3D image generating unit configured to detect feature information from the patient image and the actual surgical skill data, and generate the 3D surgical image using the feature information.

2. The surgical skill training system of claim 1, wherein the actual surgical skill data for each surgical stage comprises first surgical skill data performed during a preparation stage, second surgical skill data performed during an intermediate stage after the preparation stage, and third surgical skill data performed during a finishing stage after the intermediate stage, and
the surgical stage classifying unit is configured to extract first surgical movement information of the operating surgeon from the actual surgical skill data and classify the actual surgical skill data into at least one of the first surgical skill data, the second surgical skill data, and the third surgical skill data, based on the first surgical movement information.

3. The surgical skill training system of claim 2, wherein the image providing server further comprises:
a feature information detecting unit configured to detect the feature information for each of the first surgical skill data, the second surgical skill data, and the third surgical skill data, respectively; and
an image signal generating unit configured to generate an image signal based on the feature information, and
the 3D image generating unit is further configured to generate the 3D surgical image by combining the image signal with the patient image, and generate the 3D surgical image using a first surgical image generated based on the first surgical skill data, a second surgical image generated based on the second surgical skill data, and a third surgical image generated based on the third surgical skill data.

4. The surgical skill training system of claim 2, wherein the first surgical skill data comprises surgical instrument information, patient posture information, and surgical assistive instrument information,
the second surgical skill data comprises surgical route information and surgical movement information, and
the third surgical skill data comprises suture route information, suture method information, and residue information indicating whether the surgical instrument or surgical assistive instrument remains in a human body.

5. The surgical skill training system of claim 1, wherein the data collecting unit comprises a data collecting apparatus configured to obtain the actual surgical skill data by being worn on the operating surgeon, and
the data collecting apparatus comprises a detecting unit configured to obtain tactile information of the actual surgical skill data by using a sensor, and a photographing unit configured to obtain visual information of the actual surgical skill data by using an optical element.

6. The surgical skill training system of claim 1, wherein the user device comprises:
a communication unit configured to receive the 3D surgical image;
an interface unit configured to receive a user request of selecting the patient information or the surgical stage; and
a display unit configured to display the 3D surgical image,
wherein the patient information comprises at least one of a disease type, gender, age, height, weight, body mass index (BMI), period of illness, and underlying disease of the patient.

7. A machine learning-based surgical guide system comprising:
a data collecting unit configured to collect a plurality of pieces of actual surgical skill data on patients of a plurality of operating surgeons;
an image providing server comprising a learning model trained based on the plurality of pieces of actual surgical skill data, and a 3-dimensional (3D) image generating unit configured to generate a 3D surgical guide image based on a selected image output by the learning model; and
a user device configured to receive the 3D surgical guide image from the image providing server and display the 3D surgical guide image,
wherein the learning model is configured to classify the plurality of pieces of actual surgical skill data according to surgery item information comprising patient information and surgical stage information, and output the selected image from the plurality of pieces of actual surgical skill data according to a user input of selecting a surgical condition.

8. The machine learning-based surgical guide system of claim 7, wherein
the learning model is further configured to categorize the plurality of pieces of actual surgical skill data by using a patient information recognizing unit, a surgical instrument recognizing unit, a surgical stage classifying unit, a movement information detecting unit, and a successful rate evaluating unit.

9. The machine learning-based surgical guide system of claim 8, wherein the plurality of pieces of actual surgical skill data comprises first surgical skill data performed during a preparation stage, second surgical skill data performed during an intermediate stage after the preparation stage, and third surgical skill data performed during a finishing stage after the intermediate stage, and
the surgical stage classifying unit of the learning model is configured to classify the first surgical skill data, the second surgical skill data, and the third surgical skill data according to first surgical movement information of the plurality of operating surgeons.

10. The machine learning-based surgical guide system of claim 9, wherein
the image providing server comprises:
a feature information detecting unit configured to detect feature information from surgical skill data included in the selected image; and
an image signal generating unit configured to generate an image signal corresponding to the feature information.

11. The machine learning-based surgical guide system of claim 10, wherein the 3D image generating unit is further configured to generate the 3D surgical guide image by combining the image signal with the selected image, and generate the 3D surgical guide image using a first surgical image generated based on the first surgical skill data, a second surgical image generated based on the second surgical skill data, and a third surgical image generated based on the third surgical skill data.

12. The machine learning-based surgical guide system of claim 8, wherein the successful rate evaluating unit is configured to detect successful rates of surgeries with the respect to the plurality of pieces of actual surgical skill data and classify the plurality of pieces of actual surgical skill data into surgery success data or surgery failure data.
